# NEUE EUROPÄISCHE PATENTSCHRIFT

(11) **EP 3 374 276 B2**
(45) Veröffentlichungstag und Bekanntmachung des Hinweises auf die Entscheidung über den Einspruch: **15.05.2024**
(45) Hinweis auf die Patenterteilung: 27.11.2019
(21) Anmeldenummer: 16793775.4
(22) Anmeldetag: 21.10.2016
(51) Int. Cl.: B65D 5/42, B44B 5/00

(54) **VERPACKUNGSBEHÄLTER FÜR KENNZEICHEN-PLATINEN; VERFAHREN ZUM BETREIBEN EINER PRÄGEPRESSE UNTER VERWENDUNG DES VERPACKUNGSBEHÄLTERS UND PRÄGEPRESSE**
PACKAGING CONTAINER FOR LICENSE PLATES; METHOD FOR OPERATING AN EMBOSSING PRESS USING THE PACKAGING CONTAINER; AND EMBOSSING PRESS
RÉCIPIENT D'EMBALLAGE POUR PLAQUES D'IMMATRICULATION, PROCÉDÉ PERMETTANT DE FAIRE FONCTIONNER UNE PRESSE À ESTAMPER EN UTILISANT LE RÉCIPIENT D'EMBALLAGE ET PRESSE À ESTAMPER

(30) Priorität: 20.01.2016 DE 102016100929
(43) Veröffentlichungstag der Anmeldung: 19.09.2018
(73) Patentinhaber: Erich Utsch AG, 57080 Siegen (DE)
(72) Erfinder: WOLLENWEBER, Thomas, 57076 Siegen (DE); KÖLSCH, Jörg, 57234 Wilnsdorf (DE)
(74) Vertreter: Patentanwälte Bauer Vorberg Kayser
(86) Internationale Anmeldenummer: PCT/EP2016/075372
(87) Internationale Veröffentlichungsnummer: WO 2017/125175

(56) Entgegenhaltungen:
- EP-A1- 1 459 988
- EP-A2- 1 715 447
- EP-B1- 1 207 082
- WO-A1-2008/135921
- WO-A1-2014/170261
- DE-A1-102006 057 644
- US-B1- 6 456 729
- https://tenders.procurement.gov.ge/public/ ?go=112135&lang=en? Fotos von der Messe "Gulf Traffic 2013" in Dubai, Interne Dokumentation Messenbericht Gulf Traffic 2013"'

## Beschreibung

Offenbart wird ein Verpackungsbehälter zur Aufnahme von Kennzeichen-Platinen gemäß dem Oberbegriff des Anspruchs 1. Dabei ist jede Kennzeichen-Platine mit einer sie eindeutig identifizierenden Platinenidentifikation versehen, die optoelektronisch erfassbar ist und in der Informationen über die Kennzeichen-Platine verschlüsselt sind.

Die Erfindung betrifft ein Verfahren zum Betreiben einer Prägepresse unter Verwendung eines solchen Verpackungsbehälters und eine zugehörige Prägepresse.

Im Bereich der Herstellung von KFZ-Kennzeichenschildern sind Prägepressen bekannt, mit denen in einen Kennzeichen-Rohling eine bestimmte Kennzeichen-Legende in Form beispielsweise von Buchstaben und Zahlen eingeprägt und anschließend in einer weiteren Maschine eingefärbt wird. Die dabei verwendeten Kennzeichen-Rohlinge werden auch als Kennzeichen-Platinen bezeichnet und haben üblicherweise bereits das gewünschte Format und optional weitere Merkmale wie beispielsweise einen erhabenen umlaufenden Rand, landesspezifische Aufdrucke, Sicherheitsmerkmale, etc. Wird für ein Fahrzeug eine bestimmte Kennzeichen-Legende vergeben, wird diese individuell in eine Kennzeichen-Platine eingebracht.

In der Prägepresse wird die Legende dann erhaben in die Platine geprägt. Dazu wird eine Kennzeichen-Platine in die Prägepresse eingelegt. Die für die Prägung erforderlichen alphanumerischen Prägewerkzeuge in Form von sogenannten Klotzwerkzeugen werden von einem Bediener manuell beispielsweise in eine Schublade gelegt und zusammen mit der Platine in die Prägepresse eingeschoben. Die Prägepresse kann dann den Prägevorgang für eine bestimmte Kennzeichen-Legende durchführen. Eine solche Prägepresse in Kombination mit einer Maschine zum anschließenden Einfärben der Legende ist beispielsweise aus der EP 1 207 082 B1 bekannt.

Derartige Prägepressen können insbesondere in KFZ-Zulassungsstellen zur Anwendung kommen, bei denen ein Bediener von einer Person einen Laufzettel der KFZ-Behörde erhält, auf dem die zu prägende Kennzeichen-Legende vermerkt ist. Ein solcher Prägeauftrag kann auch über das Internet oder sonstige andere Datenverbindungen an einen Bediener einer Prägepresse übermittelt werden. Um Kennzeichen-Platinen in einem solchen Gesamtsystem zu handhaben, ist es beispielsweise bekannt, diese mit Identifikationen wie Seriennummern in Klarschrift oder auch in Form von Barcodes zu versehen.

Beim Prägevorgang kann es jedoch zu manuellen Fehlern kommen, wenn der Bediener beispielsweise nicht die korrekten Klotzwerkzeuge einlegt oder eine falsche Platine verwendet. Ferner können Manipulationen stattfinden, wenn versucht wird, eine bestimmte, von der KFZ-Behörde vergebene Legende in mehrere Kennzeichen-Platinen eingebracht wird. Bekannt ist es daher auch, einer Prägepresse einen Prägeauftrag auf elektronischem Weg zu übermitteln, wodurch die für den erforderlichen Prägevorgang erforderlichen Informationen in der Prägepresse hinterlegt werden können. Beispielsweise können einem Bediener dann verschiedene Informationen auf einem Monitor angezeigt werden, um Fehler beim Prägen zu vermeiden. Jegliche Fehler und auch Manipulationen lassen sich so jedoch nicht vollständig verhindern.

Aus der WO 2008/135921 A1 sind weiterhin Kennzeichen-Platinen bekannt, die jeweils mit einer sie eindeutig identifizierenden Platinenidentifikation versehen sind, die optoelektronisch erfassbar ist und in der Informationen über die jeweilige Kennzeichen-Platine verschlüsselt sind

Aufgabe der Erfindung es daher, eine Art der Verwindung von Kennzeichen-Platinen an einer Prägepresse vorzuschlagen, mit der sich Fehler und Manipulationversuche besser verhindem lassen.

Erfindungsgemäß wird die Aufgabe durch ein Verfahren zum Betreiben einer Prägepresse gemäß Anspruch 1 gelöst, wobei sich eine vorteilhafte Weiterbildung dieses Verfahrens aus Unteranspruch 2 und eine zugehörige Prägepresse aus dem Anspruch 3 ergeben.

Der Verpackungsbehälter dient zur Aufnahme von Kennzeichen-Platinen, wobei jede Kennzeichen-Platine mit einer sie eindeutig identifizierenden Platinenidentifikation versehen ist. Diese Platinenidentifikation ist optoelektronisch erfassbar und in ihr sind Informationen über die jeweilige Kennzeichen-Platine verschlüsselt. Erfindungsgemäß sind in dem Verpackungsbehälter wenigstens zwei dieser Kennzeichen-Platinen aufgenommen, und der Verpackungsbehälter ist mit einer Behälteridentifikation versehen, in der die jeweils in den Platinenidentifikationen der wenigstens zwei Kennzeichen-Platinen verschlüsselten Informationen wenigstens teilweise hinterlegt sind. Vorzugsweise sind diese Informationen in der Behälteridentifikation verschlüsselt hinterlegt.

Bei dem Verpackungsbehälter kann es sich um einen beliebigen Behälter bzw. eine als Behälter wirkende Umhüllung handeln, der/die sich zur Aufnahme mehrerer Kennzeichen-Platinen eignet. Dabei kann der Behälter vollständig geschlossen oder auch nur teilweise geschlossen ausgeführt sein. Beispielsweise kann er durch einen Karton, eine Umwickelung aus Folie, eine Palette, etc. gebildet sein. Mehrere Kennzeichen-Paletten mit jeweiligen Platinenidentifikationen können so in einen Karton eingebracht und der Karton mit einer Behälteridentifikation versehen werden. Mehrere Kennzeichen-Platinen können auch mit einer Folie umwickelt und die Folie mit einer Behälteridentifikation versehen werden. Das Gleiche gilt beispielsweise für eine Palette, auf der mehrere Kennzeichen-Platinen abgelegt und dort mit einer Folie umwickelt sind.

Diese Beispiele sind jedoch nicht einschränkend zu verstehen, sondern die Erfindung erstreckt sich auf jegliche Arten von Behältern oder Transportmitteln, mit denen insbesondere mehrere Kennzeichen-Platinen gemeinsam zu einer Prägepresse transportiert werden können, bevor sie dort einzeln für Prägevorgänge anhand von Prägeaufträgen für Kennzeichen-Legenden verwendet werden.

Der Verpackungsbehälter hat dabei den Vorteil, dass durch Auslesen einer einzigen Behälteridentifikation alle zulässigen Platinenidentifikationen von Kennzeichen-Platinen in einer Verpackung erfasst und beispielsweise in der Prägepresse hinterlegt werden können. Diese erfassten Platinenidentifikationen können anschließend von der Prägepresse an eine zentrale Verwaltungseinheit gesendet werden, um dieser Informationen über zu verwendende Kennzeichen-Platinen zu übermitteln. Die Prägepresse wäre dann in einem Gesamtsystem wenigstens zeitweise online, um Informationen mit anderen Komponenten des Systems auszutauschen. Ein solches System könnte wenigstens aus mehreren Prägepressen und einer zentralen Verwaltungseinheit bestehen, wobei Daten der zentralen Verwaltungseinheit dazu genutzt werden könnten, um Prägevorgänge an den einzelnen Prägepressen zu überprüfen und zu authentifizieren.

Besonders vorteilhaft kann die Erfindung jedoch eingesetzt werden, wenn eine Prägepresse auch offline arbeiten können soll, d.h. ohne Verbindung zu anderen Komponenten eines Gesamtsystems. Um Kennzeichen-Platinen anhand ihrer Platinenidentifikation zu überprüfen und einen Prägevorgang nur freizugeben, wenn es sich bei einer eingelegten Kennzeichen-Platine um eine zulässige Kennzeichen-Platine handelt, sollten an der Prägepresse Informationen über zulässige Kennzeichen-Platinen vorliegen. Die Prägepresse muss dann beispielsweise nicht mit einer zentralen Datenbank verbunden sein, in der diese Informationen für das Gesamtsystem vorgehalten werden. Dies kann vielmehr mit dem Verpackungsbehälter verwirklicht werden, in dessen Behälteridentifikation Informationen zu zulässigen Platinenidentifikationen hinterlegt sind. Werden diese Informationen an der Prägepresse ausgelesen und in der Prägepresse hinterlegt, können sie für anschließende Überprüfungen der Platinenidentifikationen von in die Prägepresse eingebrachten Kennzeichen-Platinen genutzt werden.

Bei einer optoelektronisch erfassbaren Platinenidentifikation kann es sich in der einfachsten Form beispielsweise um einen eindimensionalen Barcode handeln. Vorzugsweise wird als Platinenidentifikation einer Kennzeichen-Platine und/oder als Behälteridentifikation eines Verpackungsbehälters jedoch jeweils ein zweidimensionaler Data-Matrixcode verwendet. Ein solcher Data-Matrixcode wird im Folgenden auch verkürzt als DMC bezeichnet. Er hat den Vorteil, dass er eine sehr hohe Informationsdichte aufweist, da viele Informationen auf sehr kleinem Platz verschlüsselt werden können. Dies ist insbesondere von Vorteil, wenn eine Platinenidentifikation auf einer Kennzeichen-Platine möglichst unauffällig angebracht sein soll. Der DMC hat ferner eine hohe Datensicherheit, denn auch wenn ca. 25% der Daten zerstört oder für eine Scanvorrichtung nicht sichtbar sind, kann der DMC noch ausgelesen werden.

Um die Manipulationssicherheit zu erhöhen, sieht eine bevorzugte Ausführungsform der Erfindung vor, dass Informationen über Kennzeichen-Platinen in einer Behälteridentifikation mit einer symmetrischen und/oder asymmetrischen Verschlüsselung verschlüsselt sind. Dabei können beide Verschlüsselungsarten getrennt oder auch in Kombination angewendet werden. Die Verwendung einer asymmetrischen Verschlüsselung kann beispielsweise vorsehen, dass die Informationen in der Behälteridentifikation bei ihrer Erzeugung mit einem öffentlichen Schlüssel (public key) verschlüsselt werden. Die Informationen können dann beim Einlesen der Behälteridentifikation an der Prägepresse mit einem geheimen Schlüssel (private key) entschlüsselt werden, welcher der Prägepresse bekannt ist.

Um die Sicherheit weiter zu erhöhen, kann diese asymmetrische Verschlüsselung durch eine symmetrische Verschlüsselung ergänzt werden. Beispielsweise kann bei der Erzeugung der Behälteridentifikation ein geheimer Schlüssel erzeugt und verwendet werden, mit dem die Informationen in der Behälteridentifikation verschlüsselt werden. Dieser geheime Schlüssel wird in der Behälteridentifikation hinterlegt, so dass er nach Entschlüsselung der asymmetrischen Verschlüsselung ausgelesen werden kann, um damit die symmetrische Verschlüsselung der Informationen zu entschlüsseln.

In der Behälteridentifikation kann ferner eine Behälterkennung hinterlegt oder auch verschlüsselt sein, mit welcher der Verpackungsbehälter eindeutig identifizierbar ist. Diese Verschlüsselung kann ebenfalls eine symmetrische und/oder asymmetrische Verschlüsselung beinhalten oder die Behälterkennung ist lediglich in der Datenmatrix des Data-Matrixcodes verschlüsselt. Diese Behälterkennung kann ebenfalls zu Überprüfungszwecken genutzt werden, um beispielsweise beim Auslesen der Behälteridentifikation an der Prägepresse zu überprüfen, ob es sich um einen zulässigen Behälter handelt.

In der Platinenidentifikation einer Kennzeichen-Platine können Informationen über diese Kennzeichen-Platine mit verschiedenen Zeichenlängen enthalten sein. Ein DMC hat dann in Abhängigkeit von den in ihm dargestellten Zeichen eine entsprechende Größe. Diese Informationen über eine jeweilige Kennzeichen-Platine können insbesondere eine Seriennummer umfassen, mit welcher die Kennzeichen-Platine eindeutig im System identifizierbar ist. Ferner können Informationen wie Produktionsdaten, das Format der Platine, etc. enthalten sein. Um die Größe beispielsweise eines DMCs relativ klein zu halten, haben sich Zeichenlängen im Bereich von 10-20, insbesondere von 16 als vorteilhaft erwiesen. Es können jedoch auch jegliche anderen Zeichenlängen gewählt werden.

Dabei müssen nicht alle Zeichen für Informationen über eine Kennzeichen-Platine verwendet werden, sondern einige Zeichen können auch für andere Zwecke eingesetzt werden. Beispielsweise können einige Zeichen durch eine Hashfunktion anderer Zeichen gebildet werden. Diese Hashwerte können für Überprüfungszwecke genutzt werden, um beispielsweise eine Integritätsprüfung durchzuführen.

Erfindungsgemäß bestehen die in einer Platinenidentifikation verschlüsselten Informationen über eine Kennzeichen-Platine wenigstens aus einer ersten Zeichenfolge mit n Zeichen und einer zweiten Zeichenfolge mit m Zeichen, wobei die n Zeichen der ersten Zeichenfolge durch eine Hashfunktion in die m Zeichen der zweiten Zeichenfolge abgebildet sind. Es ist dabei vorgesehen, dass nur die n Zeichen der ersten Zeichenfolge in der Behälteridentifikation hinterlegt bzw. verschlüsselt sind. Dies hat insbesondere den Vorteil, dass die Abmessungen der Behälteridentifikation gegenüber der Verschlüsselung aller Zeichen der mehreren Platinenidentifikationen eines Verpackungsbehälters reduziert sind.

Von der Erfindung umfasst ist ferner ein Verfahren zum Betreiben einer Prägepresse, welche zum Prägen von Kennzeichen-Platinen ausgebildet ist, unter Verwendung eines Verpackungsbehälters gemäß einem oder mehreren der beschriebenen Ausführungsformen. Das Verfahren zeichnet sich durch wenigstens folgende Schritte aus:
a) Erfassen der Behälteridentifikation von dem Verpackungsbehälter durch eine Scanvorrichtung der Prägepresse;
b) Auslesen von Informationen zu den Kennzeichen-Platinen aus der erfassten Behälteridentifikation durch eine Steuereinheit der Prägepresse und Speichern dieser Informationen.

Mit diesem Verfahren können somit anhand einer Behälteridentifikation eines Verpackungsbehälters alle Platinenidentifikationen von darin enthaltenen Kennzeichen-Platinen an einer Prägepresse ausgelesen und gespeichert werden. Bevor ein Bediener Kennzeichen-Platinen aus einem Verpackungsbehälter entnehmen kann, um sie für Prägeaufträge zu verwenden, muss er daher die Behälteridentifikation des Behälters erfassen. Die so erfassten Platinenidentifikationen können auf verschiedene Arten vorteilhaft genutzt werden. Insbesondere können sie für eine Überprüfung der Zulässigkeit von Kennzeichen-Platinen verwendet werden, die dann von einem Bediener für einen Prägeauftrag in die Prägepresse eingebracht werden.

Dabei können die Informationen im Schritt b) in einer Speichereinheit der Prägepresse oder einer Komponente entfernt von der Prägepresse gespeichert werden, wobei eine wenigstens temporäre Kommunikationsverbindung zwischen der Prägepresse und dieser Komponente besteht. Die Überprüfung von Kennzeichen-Platinen, die von einem Bediener für einen Prägeauftrag in die Prägepresse eingebracht werden, kann in der ersten Ausführungsform von der Prägepresse selbst durchgeführt werden, während die Überprüfung in der zweiten Ausführungsform auch von der besagten anderen Komponente durchgeführt werden kann.

Die Erfassung einer Behälteridentifikation kann beispielsweise durch einen Handscanner erfolgen, der in Verbindung mit einer Steuereinheit der Prägepresse steht. Als Scanvorrichtung kommt bei Behälteridentifikationen in Form von Data-Matrixcodes eine Kamera zum Einsatz, die ein Bild des DMC aufnimmt. Dieses Bild wird der Steuereinheit der Prägepresse übermittelt, welche mit einer Software Informationen aus der Behälteridentifikation auslesen kann. Dabei können die bereits genannten Entschlüsselungen stattfinden, um schließlich die Informationen über alle Kennzeichen-Platinen in dem Verpackungsbehälter erhalten und speichern zu können.

Das Verfahren wird ergänzt durch die Schritte
c) Erfassen einer Platinenidentifikation von einer Kennzeichen-Platine;
d) Auslesen der Informationen über diese Kennzeichen-Platine aus der Platinenidentifikation durch die Prägepresse;
e) Vergleich der im Schritt d) ausgelesenen Informationen über diese Kennzeichen-Platine mit in der Speichereinheit der Prägepresse gespeicherten Informationen über Kennzeichen-Platinen;
f) Freigabe eines Prägevorgangs an der Prägepresse, falls der Vergleich im Schritt e) ergibt, dass die ausgelesenen Informationen über diese Kennzeichen-Platine mit in der Speichereinheit gespeicherten Informationen über Kennzeichen-Platinen übereinstimmen.

Soll eine Kennzeichen-Platine von einer Prägepresse für einen Prägeauftrag verwendet werden, muss somit zunächst in Schritt c) die Platinenidentifikation der jeweiligen Platine an der Prägepresse erfasst werden, um Informationen über die Platine auslesen zu können. Dies kann ebenfalls durch den Bediener und einen Handscanner erfolgen. Die Erfassung kann jedoch auch automatisch stattfinden, wenn eine Kennzeichen-Platine in die Prägepresse eingebracht wird. Auch hier kann als Scanvorrichtung eine Kamera zum Einsatz kommen, deren Bild eines DMC der Steuereinheit der Prägepresse übermittelt wird, um daraus Informationen über die jeweilige Kennzeichen-Platine auszulesen.

Die so ausgelesenen Informationen werden dann mit denjenigen Informationen über zulässige Kennzeichen-Platinen eines Verpackungsbehälters verglichen, die zuvor anhand einer Behälteridentifikation erfasst und in der Prägepresse hinterlegt wurden. Nur wenn dieser Vergleich ergibt, dass die ausgelesenen Informationen mit in der Speichereinheit gespeicherten Informationen über Kennzeichen-Platinen übereinstimmen, erfolgt die Freigabe eines Prägevorgangs. Anderenfalls wird der Prägevorgang blockiert, weil es sich bei der erfassten Platinenidentifikation in diesem Fall um eine unzulässige Kennzeichen-Platine handelt. Unzulässig kann die Platine beispielsweise sein, weil es sich grundsätzlich um keine zulässige Platine handelt. Dies kann der Fall sein, wenn an einer Prägepresse nur Kennzeichen-Platinen eines bestimmten Herstellers zu verwenden sind, die Kennzeichen-Platine jedoch nicht zu dieser Gruppe gehört. Unzulässig kann eine Platine auch sein, wenn es sich zwar um eine Kennzeichen-Platine eines bestimmten Herstellers handelt, diese Platine jedoch bereits einmal für einen Prägevorgang verwendet wurde bzw. bereits einmal an der Prägepresse erfasst wurde. Dies deutet auf einen Manipulationsversuch hin, der durch die Blockierung des Prägevorgangs verhindert wird.

Um anhand eines Vergleichs mit gespeicherten Informationen bereits verwendete Kennzeichen-Platinen festzustellen, müssen die Informationen von verwendeten Kennzeichen-Platinen beispielsweise in der Speichereinheit der Prägepresse entsprechend gekennzeichnet werden. Dabei bedeutet eine Verwendung einer Kennzeichen-Platine, dass ihre Platinenidentifikation einmal für einen Prägevorgang an der Prägepresse erfasst wurde. In einer Ausführungsform der Erfindung ist dann vorgesehen, dass die jeweils in der Speichereinheit der Prägepresse gespeicherten Informationen als verwendet gekennzeichnet werden, wenn in Schritt f) die Freigabe eines Prägevorgangs erfolgt. Dabei können für die Kennzeichnung verschiedene Maßnahmen getroffen werden. Beispielsweise können die betreffenden Informationen mit einer Kennzeichnung versehen werden, wobei für den Vergleich in Schritt f) nur diejenigen Informationen herangezogen werden, die keine solche Kennzeichnung aufweisen. Informationen zu noch nicht verwendeten Kennzeichen-Platinen können jedoch auch von Anfang an mit einer Kennzeichnung versehen werden, die gelöscht wird, sobald eine Kennzeichen-Platine verwendet wurde. Für den Vergleich in Schritt f) werden dann nur diejenigen Informationen herangezogen, die noch eine solche Kennzeichnung aufweisen. Auch ein vollständiges Löschen von Informationen zu verwendeten Kennzeichen-Platinen ist möglich, so dass bei dem Vergleich in Schritt f) nur diejenigen Informationen herangezogen werden, die noch vorhanden sind, d.h. deren zugehörige Kennzeichen-Platinen noch nicht verwendet wurden.

In Schritt b) vor dem Speichern der Informationen wird ebenfalls eine Hashfunktion aus den n Zeichen der ersten Zeichengruppe der ausgelesenen Informationen über die Kennzeichen-Platine erzeugt und gespeichert. Die so erhaltenen Hashwerte können dann für eine Integritätsprüfung mit den Hashwerten aus einer von einer Kennzeichen-Platine ausgelesenen Platinenidentifikation verglichen werden.

Das Verfahren kann an einer Prägepresse durchgeführt werden, die entsprechend ausgebildet ist. Insbesondere kann eine solche Prägepresse somit ausgebildet sein zum
- Erfassen der Behälteridentifikation von dem Verpackungsbehälter durch eine Scanvorrichtung der Prägepresse;
- Auslesen von Informationen zu den Kennzeichen-Platinen aus der erfassten Behälteridentifikation durch eine Steuereinheit der Prägepresse und Speichern dieser Informationen in einer Speichereinheit der Prägepresse.

Ergänzend ist die Prägepresse ausgebildet zum
Erfassen einer Platinenidentifikation von einer Kennzeichen-Platine;
Auslesen der Informationen über diese Kennzeichen-Platine aus der Platinenidentifikation durch die Prägepresse;
Vergleich der ausgelesenen Informationen über diese Kennzeichen-Platine mit in der Speichereinheit der Prägepresse gespeicherten Informationen über Kennzeichen-Platinen;
Freigabe eines Prägevorgangs an der Prägepresse, falls der Vergleich ergibt, dass die ausgelesenen Informationen über diese Kennzeichen-Platine mit in der Speichereinheit gespeicherten Informationen über Kennzeichen-Platinen übereinstimmen.

In einer weiteren Ausführungsform ist die Prägepresse ausgebildet, um die jeweils in der Speichereinheit der Prägepresse gespeicherten Informationen als verwendet zu kennzeichnen, wenn die Freigabe eines Prägevorgangs erfolgt. Ferner kann sie dazu ausgebildet sein, vor dem Speichern der Informationen aus der Erfassung einer Behälteridentifikation eine Hashfunktion aus den n Zeichen der ersten Zeichengruppe der ausgelesenen Informationen über die Kennzeichen-Platine zu erzeugen und zu speichern.

Eine solche Prägepresse ist sehr sicher gegen Manipulationen, dabei von einem Nutzer jedoch einfach zu bedienen. Ferner kann sie auch offline ohne Verbindung zu Datenbanken betrieben werden, in denen Informationen über zulässige Kennzeichen-Platinen hinterlegt sind, da die hierfür notwendigen Informationen über das Einlesen der Behälteridentifikation eines Verpackungsbehälters für mehrere Kennzeichen-Platinen an der Prägepresse bereitgestellt werden können.

Weitere Vorteile, Besonderheiten und zweckmäßige Weiterbildungen der Erfindung ergeben sich aus den Unteransprüchen und der nachfolgenden Darstellung bevorzugter Ausführungsbeispiele anhand der Abbildungen.

Von den Abbildungen zeigt:
Fig. 1 : eine Darstellung der Vorderseite einer Kennzeichen-Platine;
Fig. 2 : eine Darstellung der Rückseite einer Kennzeichen-Platine gemäß Fig. 1 mit einem Data-Matrixcode;
Fig. 3 : einen schematischen Querschnitt durch eine Ausführungsform eines Verpackungsbehälters;
Fig. 4 : eine Draufsicht auf einen Verpackungsbehälter mit einem Data-Matrixcode;
Fig. 5 : einen schematischen Querschnitt durch eine Ausführungsform einer Prägepresse; und
Fig. 6 : das Einschieben einer Kennzeichen-Platine in eine Prägepresse gemäß Fig. 5 .

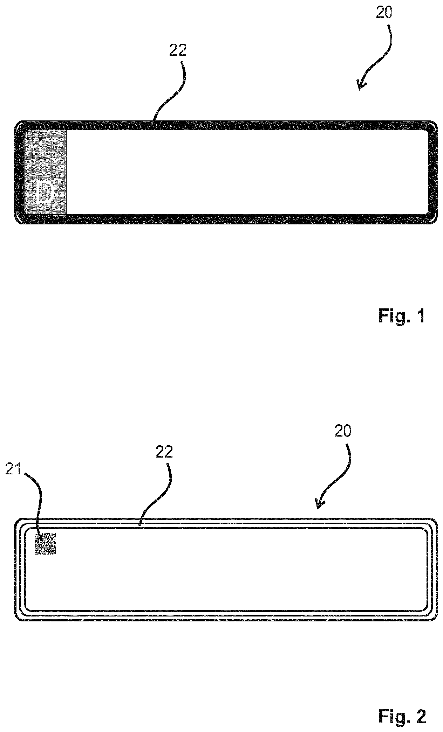

Die in Fig. 1 dargestellte Vorderseite einer Kennzeichen-Platine 20 zeigt beispielhaft eine mögliche Ausführungsform einer solchen Platine. Sie hat in diesem Ausführungsbeispiel eine langgestreckte, rechteckige Form. Es sind jedoch auch andere Formate mit anderen Seitenverhältnissen möglich. Die Platine 20 besteht aus einem Blechstreifen, wobei als Material beispielsweise eine Aluminiumlegierung gewählt werden kann. In diesen Blechstreifen ist bereits in umlaufender Rand 22 eingeprägt, der auf der Vorderseite erhaben ausgebildet und mit Farbe bedruckt ist. Im linken Bereich der Platine sind weitere Farbaufdrucke aufgebracht, die jedoch ebenfalls nur beispielhaft zu verstehen sind. Insgesamt kann die Kennzeichen-Platine landestypisch unterschiedliche Gestaltungen aufweisen.

Auf der in Fig. 2 dargestellten Rückseite der Kennzeichen-Platine 20 ist der eingeprägte Rand von hinten ersichtlich und das aufgedruckte Identifikationsmerkmal 21. Hierbei handelt es sich um einen zweidimensionalen Data-Matrixcode (DMC) an sich bekannter Art. Dieser DMC ist beispielsweise in einer Ecke der Platine 20 aufgebracht und kann mit einer Scanvorrichtung erfasst werden, um die darin verschlüsselten Daten auszulesen. Der DMC kann dabei insbesondere aufgedruckt oder eingelasert sein.

In dem Data-Matrixcode 21 einer Kennzeichen-Platine 20, der im Folgenden auch als Plate-DMC bezeichnet wird, können verschiedene Informationen zu der jeweiligen Kennzeichen-Platine verschlüsselt sein. Dabei handelt es sich insbesondere um eine Seriennummer, mit welcher die Kennzeichen-Platine in einem Gesamtsystem eindeutig identifizierbar ist. Ferner können Informationen wie Produktionsdaten, das Format oder ähnliche Angaben enthalten sein. Der Plate-DMC 21 ist mit einer Scanvorrichtung wie einer Kamera erfassbar, wobei die verschlüsselten Informationen mit einer entsprechenden Software aus dem Bild der Kamera auslesbar sind.

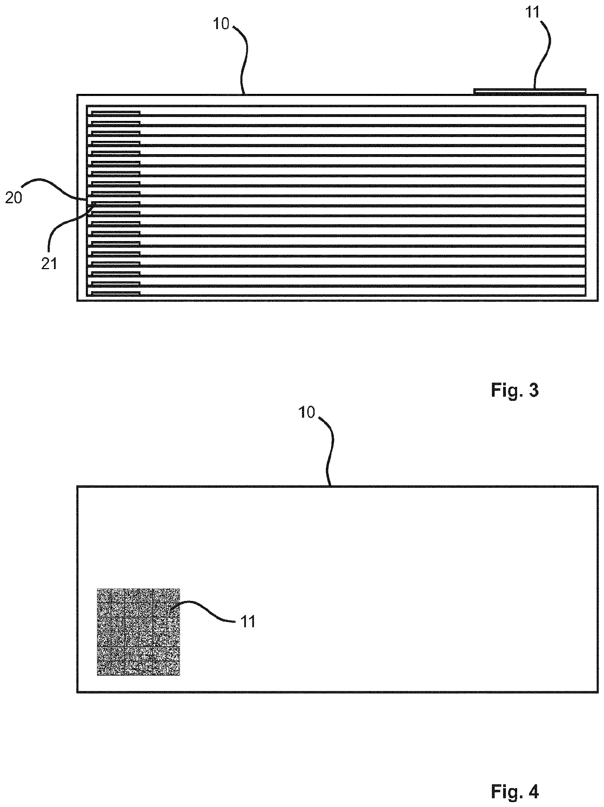

Die Darstellung der Fig. 3 zeigt schematisch einen Querschnitt durch eine Ausführungsform eines Verpackungsbehälters 10. Hierbei handelt es sich um einen einfachen quaderförmigen Behälter beispielsweise in Form eines Kartons. In diesen Karton ist eine Vielzahl von Kennzeichen-Platinen 20 eingebracht, die übereinander gestapelt sind. Die Anzahl der Platinen richtet sich nach der Aufnahmekapazität des Behälters, dem maximal möglichen Gewicht oder anderen Kriterien, so dass die Darstellung von neunzehn übereinander gestapelten Platinen 20 in dem Verpackungsbehälter 10 der Fig. 3 nur beispielhaft ist.

Jede Kennzeichen-Platine 20 ist dabei mit einer Platinenidentifikation versehen, wie sie der Fig. 2 zu entnehmen und in der Darstellung der Fig. 3 nur schematisch angedeutet ist. Der Verpackungsbehälter 10 ist hingegen mit einer Behälteridentifikation 11 versehen, die dem Verpackungsbehälter vorzugsweise fest zugeordnet ist. Ein solcher Box-DMC ist der Draufsicht eines Verpackungsbehälters 10 der Fig. 4 zu entnehmen. Hierbei kann es sich beispielsweise um einen Aufkleber mit einem aufgedruckten Code, insbesondere Data-Matrixcode, handeln. Es kann sich jedoch beispielsweise auch um einen RFID-Transponder handeln, der auf den Behälter aufgeklebt ist. Die Behälteridentifikation 11 kann dem Behälter 10 auch auf andere Arten fest zugeordnet sein. Beispielsweise kann es sich um ein anhängendes Schild, einen direkt aufgedruckten DMC, einen beigelegten Zettel, etc. handeln.

In der Behälteridentifikation 11 sind die Informationen zu den jeweiligen Kennzeichen-Platinen 20 in diesem Verpackungsbehälter 10 wenigstens teilweise hinterlegt, so dass diese ausgelesen werden können. Dabei erfolgt die Hinterlegung vorzugsweise verschlüsselt, wobei geeignete Verfahren angewendet und je nach angestrebter Sicherheitsstufe auch miteinander kombiniert werden können. Beim Auslesen der Informationen erfolgt dann eine entsprechende Entschlüsselung. Dies kann beispielsweise an einer Prägepresse erfolgen, an welcher die Kennzeichen-Platinen 20 aus einem Verpackungsbehälter 10 verwendet werden sollen.

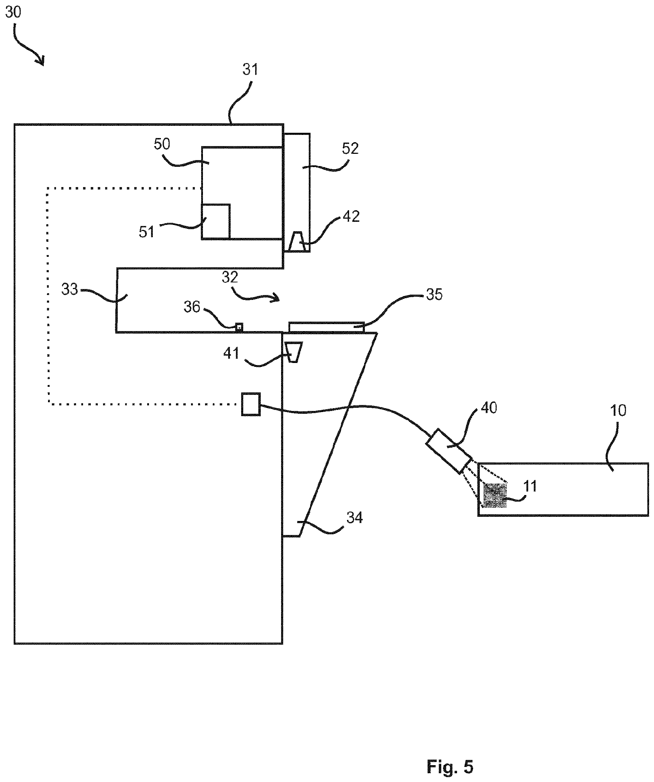

Der in Fig. 5 gezeigten schematischen Darstellung ist eine beispielhafte Ausführungsform einer Prägepresse 30 zu entnehmen, in der eine Kennzeichen-Platine 20 geprägt und dabei mit einer Legende versehen werden kann. Dabei handelt es sich vorzugsweise um ein relativ kompaktes Standgerät mit einem Gehäuse 31, in dem die wesentlichen Komponenten zum Prägen der Kennzeichen-Platinen untergebracht sind. Diese Komponenten sind nicht im Detail dargestellt, sind dem Fachmann jedoch bekannt und können ohne Weiteres je nach den gestellten Anforderungen vorgesehen werden. Insbesondere handelt es sich dabei um einen oder mehrere Pressstempel, mit denen Prägewerkzeuge in die Oberfläche einer Platine gepresst werden können.

Etwa auf Hüfthöhe eines Nutzers weist das Gehäuse 31 eine Gehäuseöffnung 32 auf, durch welche Kennzeichen-Platinen in einen schematisch angedeuteten Prägeraum 33 innerhalb des Gehäuses 31 einbringbar sind. Innerhalb dieses Prägeraums 31 findet der Prägevorgang statt. Unterhalb der Gehäuseöffnung 32 ist ein nach außen vorspringender Vorbau 34 vorgesehen, der vor der Gehäuseöffnung 32 eine Auflagefläche bildet, auf der Kennzeichen-Platinen abgelegt werden können, bevor sie in den Prägeraum 33 eingebracht werden. Insbesondere ist diese Auflagefläche als Schublade 35 ausgeführt, die horizontal verschiebbar ist. Eine Kennzeichen-Platine kann zusammen mit den entsprechenden Prägewerkzeugen auf der Schublade 35 vorbereitet und dann in die Gehäuseöffnung 32 eingeschoben werden. Beispielsweise können für einen Prägeauftrag mehrere Prägewerkzeuge in die Schublade 35 eingelegt und eine Kennzeichen-Platine darüber gelegt werden. Nach dem Prägevorgang innerhalb des Prägeraums 33 wird das geprägte Kennzeichen auf der Schublade 35 aus der Prägepresse 30 gezogen und kann entnommen werden, um die erhaben geprägte Legende einzufärben.

Oberhalb der Gehäuseöffnung 32 ist eine Funktionseinheit 52 angebracht. Diese kann in das Gehäuse 32 integriert oder außen an dem Gehäuse 31 angebracht sein, wie es beispielsweise die Ausführungsform der Fig. 5 vorsieht. Diese Funktionseinheit 52 ist vorzugsweise als Bedien- und Anzeigeeinheit ausgebildet, wobei hierzu insbesondere eine Touchscreen verwendet werden kann. Über die Funktionseinheit 52 kann ein Bediener die Prägepresse 30 steuern, ihm können Informationen zu Prägeaufträgen angezeigt werden und auch Fehlermeldungen können auf diesem Monitor angezeigt werden.

Die Funktionseinheit 52 steht in Verbindung mit einer Steuereinheit 50 der Prägepresse 30. Diese Steuereinheit 50 umfasst eine Speichereinheit 51. Die Steuereinheit kann beispielsweise über eine nicht dargestellte Kommunikationsleitung mit weiteren Komponenten eines Gesamtsystems verbunden sein. Dazu können eine zentrale Verwaltungseinheit, Auftragskomponenten zur Erzeugung von Prägeaufträgen, etc. zählen. In Verbindung mit der Steuereinheit 50 steht ferner wenigstens eine Scanvorrichtung 40 der Prägepresse, mit der eine Behälteridentifikation 11 von einem Verpackungsbehälter 10 erfasst werden kann. Das so erfasste Bild einer Behälteridentifikation 11 kann dann für ein Auslesen von Informationen an die Steuereinheit 50 der Prägepresse 30 übermittelt werden. Diese Informationen können in der Speichereinheit 51 gespeichert und/oder einer zentralen Verwaltungseinheit übermittelt werden.

Bei der Scanvorrichtung 40 handelt es sich beispielsweise um einen Handscanner mit einer Kamera und einem Kabel, der von einem Bediener in die Nähe der Behälteridentifikation 11 eines Verpackungsbehälters 10 gebracht werden kann. Die Scanvorrichtung kann jedoch auch fest in die Prägepresse 30 integriert sein, so dass der Bediener den Verpackungsbehälter vor die Scanvorrichtung halten muss. Dies kann bei schweren Verpackungsbehältern nachteilig sein, so dass ein frei beweglicher Handscanner für diesen Fall vorteilhafter ist. Es können jedoch auch beide Varianten in einer Prägepresse 30 realisiert sein.

Ergänzend können weitere Scanvorrichtungen vorgesehen sein, um Platinenidentifikationen von in die Prägepresse 30 eingebrachten Kennzeichen-Platinen erfassen zu können. Die Ausführungsform einer Prägepresse gemäß der Fig. 5 sieht beispielsweise eine weitere Scanvorrichtung 42 an der Unterseite der Funktionseinheit 52 vor. Hierbei handelt es sich vorzugsweise um eine Kamera. Mit dieser Scanvorrichtung 42 kann eine Platinenidentifikation von der Oberseite einer Kennzeichen-Platine erfasst werden, wenn diese auf die Schublade 35 aufgelegt wird. Eine weitere Scanvorrichtung (Kamera) 41 im Vorbau 34 kann hingegen dazu genutzt werden, um Platinenidentifikationen auf der Unterseite von Kennzeichen-Platinen zu erfassen.

Die dargestellten Scanvorrichtungen sind jedoch nur beispielhaft zu verstehen und es können auch jegliche andere Anordnungen und Ausführungsformen von Scanvorrichtungen vorgesehen werden, um Platinenidentifikationen von Kennzeichen-Platinen zu erfassen. Auch der im Folgenden beschriebene vorteilhafte Auslösemechanismus muss nicht zwingend gegeben und so ausgeführt sein.

Um die automatisierte Erfassung einer Platinenidentifikation auszulösen, kann nämlich ein Auslösemechanismus vorgesehen sein, der von der Bewegung der Kennzeichen-Platine selbst oder von der Schublade 35 ausgelöst werden kann. Beispielsweise kann hinter der Gehäuseöffnung 32 ein Auslösemechanismus 36 ausgebildet sein. Hierbei kann es sich beispielsweise um einen mechanischen Schalter oder eine Lichtschranke handeln. Im Ausführungsbeispiel der Fig. 5 ist beispielsweise eine Lichtschranke 36 auf Höhe der Schublade 35 angebracht. Sobald die Schublade 35 mit einer darauf befindlichen Kennzeichen-Platine die Lichtschranke 36 passiert, löst der Auslösemechanismus die Erfassung der Platinenidentifikation aus. Dabei können beide Scanvorrichtungen 41 und 42 aktiviert werden, wobei nur das Bild derjenigen Scanvorrichtung verwendet wird, auf der sich eine Platinenidentifikation befindet. Es kann jedoch auch vorgesehen sein, das die Prägepresse über Informationen verfügt, auf welcher Seite einer zu prägenden Kennzeichen-Platine sich eine Platinenidentifikation befindet. Auf diese Weise kann die Prägepresse 30 entscheiden, welche Scanvorrichtung ausgelöst wird. Diese Information über die Position eines Identifikationsmerkmals kann beispielsweise in einem Prägeauftrag enthalten sein.

Werden nur Kennzeichen-Platinen mit Identifikationsmerkmalen auf einer festgelegten Seite der Platinen verwendet, d.h. nur auf der Vorder- oder Rückseite, kann die Erfindung auch mit einer einzigen Scanvorrichtung auf der entsprechenden Seite durchgeführt werden.

Der Auslösemechanismus 36 kann insbesondere so ausgestaltet sein, dass er erst auslöst, wenn die Kennzeichen-Platine bereits teilweise in die Gehäuseöffnung 32 geschoben wurde. Ferner befindet sich der Auslösemechanismus 36 vorzugsweise innerhalb des Gehäuses 31, insbesondere an einer schwer zugänglichen Position, um möglichst auch Manipulationsversuche im Bereich des Auslösemechanismus zu verhindern.

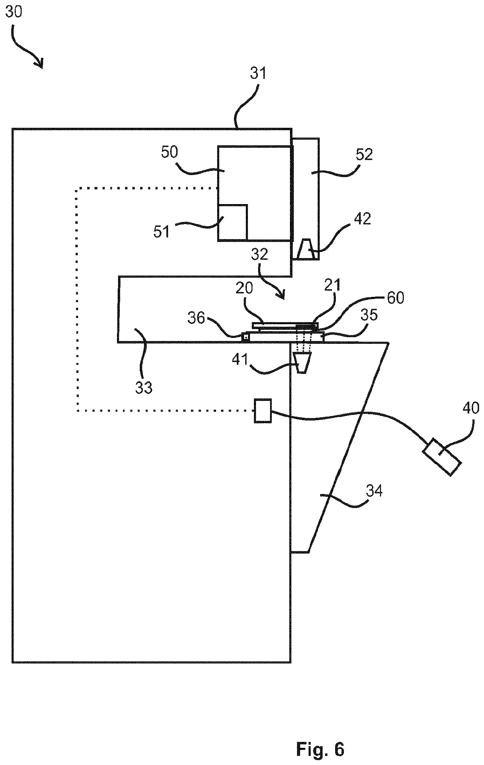

Fig. 6 zeigt beispielsweise den Zustand beim Einschieben der Schublade 35 in die Gehäuseöffnung 32. Auf der Schublade 35 befinden sich eine Kennzeichen-Platine 20 mit einer unterseitigen Platinenidentifikation 21 und Prägewerkzeuge 60, welche entsprechend einer einzuprägenden Legende ausgewählt sind. Die vordere Seite der Schublade 35 kommt in den Bereich des Auslösemechanismus 36. In dem Moment befindet sich die Platinenidentifikation 21 oberhalb der Scanvorrichtung 41, so dass diese von unten ein Bild der Platinenidentifikation 21 aufnehmen kann.

Eine solche Prägepresse 30 kann dazu verwendet werden, das erfindungsgemäße Verfahren durchzuführen. Werden die Informationen zu Kennzeichen-Platinen, welche zulässige Platinen für eine Verwendung an der Prägepresse darstellen, aus einer Behälteridentifikation ausgelesen und beispielsweise in der Speichereinheit 51 der Prägepresse 30 hinterlegt, können diese mit Informationen aus Platinenidentifikationen verglichen werden, die von Kennzeichen-Platinen ausgelesen werden, die in die Prägepresse 30 eingeschoben werden. Ergibt ein solcher Datenvergleich, dass es sich bei einer eingeschobenen Kennzeichen-Platine nicht um eine zulässige Platine handelt, kann der Prägevorgang blockiert bzw. nicht freigegeben werden.

### Bezugszeichenliste:

- 10: Verpackungsbehälter, Karton
- 11: Behälteridentifikation, Data-Matrixcode, Box-DMC
- 20: Kennzeichen-Platine, Platine
- 21: Platinenidentifikation, Data-Matrixcode, Plate-DMC
- 22: Rand
- 30: Prägepresse
- 31: Gehäuse
- 32: Gehäuseöffnung
- 33: Prägeraum
- 34: Vorbau
- 35: Schublade
- 36: Auslösemechanismus, Lichtschranke, Schalter
- 40: Scanvorrichtung, Handscanner
- 41,42: Scanvorrichtung, Kamera
- 50: Steuereinheit
- 51: Speichereinheit
- 52: Funktionseinheit, Monitor, Touchscreen
- 60: Prägewerkzeug, Klotzwerkzeug

## Patentansprüche

1. Verfahren zum Betreiben einer Prägepresse (30), welche zum Prägen von Kennzeichen-Platinen (20) ausgebildet ist, unter Verwendung eines Verpackungsbehälters (10) zur Aufnahme von Kennzeichen-Platinen (20), wobei jede Kennzeichen-Platine (20) mit einer sie eindeutig identifizierenden Platinenidentifikation (21) versehen ist, die optoelektronisch erfassbar ist und in der Informationen über die Kennzeichen-Platine (20) verschlüsselt sind, wobei in dem Verpackungsbehälter (10) wenigstens zwei Kennzeichen-Platinen (20) aufgenommen sind, und der Verpackungsbehälter (10) mit einer Behälteridentifikation (11) versehen ist, in der die jeweils in den Platinenidentifikationen (21) der wenigstens zwei Kennzeichen-Platinen (20) verschlüsselten Informationen wenigstens teilweise hinterlegt sind, **gekennzeichnet durch** wenigstens folgende Schritte:
a. Erfassen der Behälteridentifikation (11) von dem Verpackungsbehälter (10) durch eine Scanvorrichtung (40) der Prägepresse (30);
b. Auslesen von Informationen zu den Kennzeichen-Platinen (20) aus der erfassten Behälteridentifikation (11) durch eine Steuereinheit der Prägepresse (30) und Speichern dieser Informationen, wobei die Informationen in Schritt b) in einer Speichereinheit (51) der Prägepresse (30) gespeichert werden, wobei die in einer Platinenidentifikation (21) verschlüsselten Informationen über eine Kennzeichen-Platine (20) wenigstens aus einer ersten Zeichenfolge mit n Zeichen und einer zweiten Zeichenfolge mit m Zeichen bestehen, wobei die n Zeichen der ersten Zeichenfolge durch eine Hashfunktion in die m Zeichen der zweiten Zeichenfolge abgebildet sind wobei bei dem Verpackungsbehälter (10) nur die n Zeichen der ersten Zeichenfolge in der Behälteridentifikation (11) hinterlegt sind und in Schritt b) vor dem Speichern der Informationen eine Hashfunktion aus den n Zeichen der ersten Zeichengruppe der ausgelesenen Informationen über die Kennzeichen-Platine (20) erzeugt und gespeichert wird;
c. Erfassen einer Platinenidentifikation (21) von einer Kennzeichen-Platine (20);
d. Auslesen der Informationen über diese Kennzeichen-Platine (20) aus der Platinenidentifikation (21) durch die Prägepresse (30);
e. Vergleich der im Schritt d) ausgelesenen Informationen über diese Kenn-zeichen-Platine (20) mit in der Speichereinheit (51) der Prägepresse (30) gespeicherten Informationen über Kennzeichen-Platinen (20);
f. Freigabe eines Prägevorgangs an der Prägepresse (30), falls der Vergleich im Schritt e) ergibt, dass die ausgelesenen Informationen über diese Kennzeichen-Platine (20) mit in der Speichereinheit (51) gespeicherten Informationen über Kennzeichen-Platinen (20) übereinstimmen.

2. Verfahren nach Anspruch 1, **dadurch gekennzeichnet, dass** die jeweils in der Speichereinheit (51) der Prägepresse (30) gespeicherten Informationen als verwendet gekennzeichnet werden, wenn in Schritt f) die Freigabe eines Prägevorgangs erfolgt.

3. Prägepresse (30) zum Prägen von Kennzeichen-Platinen (20) unter Verwendung eines Verpackungsbehälters (10) zur Aufnahme von Kennzeichen-Platinen (20), wobei jede Kennzeichen-Platine (20) mit einer sie eindeutig identifizierenden Platinenidentifikation (21) versehen ist, die optoelektronisch erfassbar ist und in der Informationen über die Kennzeichen-Platine (20) verschlüsselt sind, wobei in dem Verpackungsbehälter (10) wenigstens zwei Kennzeichen-Platinen (20) aufgenommen sind, und der Verpackungsbehälter (10) mit einer Behälteridentifikation (11) versehen ist, in der die jeweils in den Platinenidentifikationen (21) der wenigstens zwei Kennzeichen-Platinen (20) verschlüsselten Informationen wenigstens teilweise hinterlegt sind, **dadurch gekennzeichnet, dass** sie zur Durchführung des Verfahrens gemäß Anspruch 1 oder 2 ausgebildet ist.

## Claims

1. A method for operating an embossing press (30) configured for embossing number plate panels (20), using a packing container (10) for accommodating number plate panels (20), wherein each number plate panel (20) is provided with a panel identification means (21) which unambiguously identifies it, which can be detected optoelectronically and in which information about the number plate panel (20) is encrypted, wherein at least two number plate panels (20) are accommodated in the packing container (10), and the packing container (10) is provided with a container identification means (21) in which the information respectively encrypted in the panel identification means (21) of the at least two number plate panels (20) is recorded at least partially, **characterized by** at least the following steps:
a) detecting the container identification means (11) of the packing container (10) by means of a scanning device (40) of the embossing press (30);
b) reading out information concerning the number plate panels (20) from the detected container identification means (11) by means of a control unit of the embossing press (30) and storing this information, wherein the information in step b) is stored in a storage unit (51) of the embossing press (30), wherein the information about a number plate panel (20) encrypted in a panel identification means (21) at least consists of a first string with n characters and a second string with m characters, wherein the n characters of the first string are mapped by a hash function to the m characters of the second string, wherein in the packing container (10) only the n characters of the first string are recorded in the container identification means (11) and in step b) prior to storing the information a hash function is generated from the n characters of the first group of characters of the read-out information about the number plate panel (20) and stored;
c) detecting a panel identification means (21) from a number plate panel (20);
d) reading out the information about this number plate panel (20) from the panel identification means (21) by means of the embossing press (30);
e) comparing the information about this number plate panel (20) read out in step d) with information about number plate panels (20) stored in the storage unit (51) of the embossing press (30);
f) enabling an embossing process on the embossing press (30) if the comparison in step e) shows that the read-out information about this number plate panel (20) matches information about number plate panels (20) stored in the storage unit (51).

2. The method according to claim 1,
**characterized in that** the information respectively stored in the storage unit (51) of the embossing press (30) is labelled as used if an embossing process is enabled in step f).

3. An embossing press (30) for embossing number plate panels (20) using a packing container (10) for accommodating number plate panels (20), wherein each number plate panel (20) is provided with a panel identification means (21) which unambiguously identifies it, which can be detected optoelectronically and in which information about the number plate panel (20) is encrypted, wherein at least two number plate panels (20) are accommodated in the packing container (10), and the packing container (10) is provided with a container identification means (11) in which the information respectively encrypted in the panel identification means (21) of the at least two number plate panels (20) is recorded at least partially, **characterized in that** it is configured for carrying out the method according to claims 1 or 2.

## Revendications

1. Procédé pour faire fonctionner une presse à estamper (30) qui est conçue pour estamper des plaques d'immatriculation (20) en utilisant un récipient d'emballage (10), destiné à recevoir des plaques d'immatriculation (20), chaque plaque d'immatriculation (20) étant munie d'une identification de plaque (21) qui l'identifie de manière univoque et qui peut être détectée de manière optoélectronique et dans laquelle sont cryptées des informations relatives à la plaque d'immatriculation (20), au moins deux plaques d'immatriculation (20) étant reçues dans le récipient d'emballage (10) et le récipient d'emballage (10) étant pourvu d'une identification de récipient (21) dans laquelle les informations cryptées respectivement dans les identifications de plaque (21) desdites au moins deux plaques d'immatriculation (20) sont au moins partiellement stockées,
**caractérisé par** au moins les étapes suivantes consistant à:
a. détecter l'identification de récipient (11) du récipient d'emballage (10) au moyen d'un dispositif de balayage (40) de la presse à estamper (30) ;
b. lire des informations relatives aux plaques d'immatriculation (20) à partir de l'identification de récipient (11) détectée, par l'intermédiaire d'une unité de commande de la presse à estamper (30), et stocker lesdites informations, les informations étant stockées à l'étape b) dans une unité de stockage (51) de la presse à estamper (30), les informations cryptées dans une identification de plaque (21) relatives à une plaque d'immatriculation (20) consistant dans au moins une première séquence pourvue de n caractères et dans une deuxième séquence pourvue de m caractères, les n caractères de la première séquence étant reproduits par une fonction de hachage dans les m caractères de la deuxième séquence et sur le récipient d'emballage (10) seuls les n caractères de la première séquence étant stockés dans l'identification de récipient (11) et à l'étape b), avant le stockage des informations, une fonction de hachage étant générée à partir des n caractères du premier groupe de caractères des informations lues relatives à la plaque d'immatriculation (20) et stockée ;
c. détecter une identification de plaque (21) d'une plaque d'immatriculation (20) ;
d. lire les informations relatives à cette plaque d'immatriculation (20) à partir de l'identification de plaque (21) par la presse à estamper (30) ;
e. comparer les informations relatives à ladite plaque d'immatriculation (20) qui sont lues à l'étape d) à des informations relatives à des plaques d'immatriculation (20) qui sont stockées dans l'unité de mémoire (51) de la presse à estamper (30) ;
f. libérer une opération d'estampage sur la presse à estamper (30) s'il ressort de la comparaison à l'étape e) que les informations lues relatives à ladite plaque d'immatriculation (20) correspondent à des informations relatives à des plaques d'immatriculation (20) qui sont stockées dans l'unité de stockage (51).

2. Procédé selon la revendication 1,
**caractérisé en ce que** les informations stockées chacune dans l'unité de mémoire (51) de la presse à estamper (30) sont marquées comme étant utilisées lorsque la libération d'une opération d'estampage a lieu à l'étape f).

3. Presse à estamper (30), destinée à estamper des plaques d'immatriculation (20) en utilisant un récipient d'emballage (10) destiné à recevoir des plaques d'immatriculation (20), chaque plaque d'immatriculation (20) étant munie d'une identification de plaque (21) qui l'identifie de manière univoque et qui peut être détectée de manière optoélectronique et dans laquelle sont cryptées des informations relatives à la plaque d'immatriculation (20), au moins deux plaques d'immatriculation (20) étant reçues dans le récipient d'emballage (10) et le récipient d'emballage (10) étant pourvu d'une identification de récipient (21) dans laquelle les informations cryptées respectivement dans les identifications de plaque (21) desdites au moins deux plaques d'immatriculation (20) sont au moins partiellement stockées, **caractérisé en ce qu'**il est adapté pour mettre en oeuvre le procédé selon la revendication 1 ou 2.
